# EUROPEAN PATENT SPECIFICATION

(11) **EP 2 155 049 B1**
(45) Date of publication and mention of the grant of the patent: **26.03.2014**
(21) Application number: 07847657.9
(22) Date of filing: 03.12.2007
(51) Int. Cl.: A61B 5/00, A61M 21/00

(54) **SLEEPING MODE ACCESSORY**
SCHLAFMODUS-ZUBEHÖR
ACCESSOIRE EN MODE VEILLE

(30) Priority: 08.06.2007 US 760420
(43) Date of publication of application: 24.02.2010
(73) Proprietor: Sony Ericsson Mobile Communications AB, 221 88 Lund (SE)
(72) Inventor: LINDBÄCK, Maria, S-222 40 Lund (SE); PANTORP, Rikard, S-238 32 Oxie (SE)
(74) Representative: Valea AB
(86) International application number: PCT/EP2007/063146
(87) International publication number: WO 2008/148433

(56) References cited:
- EP-A- 0 872 255
- EP-A- 1 655 051
- WO-A-01/37914
- WO-A-2006/050512
- WO-A-2006/054210
- WO-A-2006/090371
- WO-A-2006/105085
- US-A- 5 740 812
- US-A1- 2005 070 815
- US-A1- 2006 224 046
- US-A1- 2007 113 725

## Description

The present invention is related to measurement accessories for portable units and in particular to the interaction between a measurement accessory and a main unit.

### BACKGROUND OF THE INVENTION

Presently, several systems interacting with a user in order to aid the user in reaching a certain physiological state or aiding the user in order to achieve certain goals related to physical exercise are known.

As an example, biofeedback devices are on the market, which monitor the physiological state of the user via sensors and use music or sounds as feedback to the user in order to facilitate the user falling into the sleep state.

One drawback of such systems is that they are usually bulky and expensive and most of them are not portable either. One other drawback is that they are basically constructed to perform only function, i.e. the biofeedback.

Other feedback devices, such as the one disclosed in WO2006/050512 describe a method and a device for sonification of physiological data, where the heart rate, muscle tension, blood flow and other parameters are used to influence the tempo of the music or sound played or to spur the user to increase the physical training rate when performing a specific exercise, for example.

While this device provides the desired portability, the user is not able to influence the state of the portable feedback unit by means of his or her physiological state other than changing the tempo of the played music or sound.

Apart from biofeedback and portable feedback systems, portable measurement devices monitoring physiological parameters, such the already mentioned heart rate, muscle tension, pulse, temperature and other parameters also are known today.

As an example, the Japanese patent application JP2005034547 discloses a health care system for measuring and controlling health care data of a person, where the system consists of a watch comprising a sensing part for measuring physiological parameters, a receiving part comprising a cell phone and a remote displaying part for displaying the measured data on a host computer received from the cell phone.

Another example of a portable measuring device is the heart rate device described in US2005/0256416, where a heart rate sensor is integrated into a wrist watch and where the status of the heart rate is displayed using different colors. Additionally, the wrist watch may also indicate incoming calls to the ceil phone.

A drawback of both the system disclosed by JP2005034547 and the heart rate device in US2005/0256416 is the lack of controlling function from the monitoring system to the user of the monitoring system. Also, it is unclear whether the physiological data registered may have any effect on the state of the measurement system or device itself.

US 2007/113725 concerns the use of music to influence a person's performance in a physical workout. A computing device receives and analyzes data indicating current physiology and movement of the user in order to provide a music piece that will influence the user to speed up, slow down, or maintain current pace so to achieve a desired exercise performance level. Information specific to the user may be considered in providing the music piece.

US 2005/070815 discloses a conscious sedation system comprising a controller which generates an audio request for a predetermined response from a patient a response testing apparatus including a request assembly and a response assembly. The request assembly communicates to the patient the request generated by the controller and the response assembly is used by the patient to generate the response and communicates the response to the controller, wherein the controller is programmed to generate a request and to analyze the patient's response to the request and determine and record a baseline audio stimulus for the patient and after determining the baseline audio stimulus, the controller generates a request based upon the baseline stimulus. A method for using the automated audio calibration for a conscious sedation system is also disclosed.

WO 2006/050512 describes systems and methods for sonification, user influence through sound, and biofeedback, sonification of motion and physiological parameters during physical exercise and the use of music and sound in order to influence the emotional, psychological and physiological state of an exerciser, and the use of sonification and influence in a feedback loop to create a particular exercise experience for a user. A digital music player comprises physiological sensors, a controller, a user interface, a music decoder, a music playback modulator, and an audio data store. The user interface allows a user to specify target values for the physiological sensors as a function of time, the controller selects a playlist of audio data based on the target values, the music decoder decodes the audio data in a sequence corresponding to the playlist, and the music playback modulator causes the sequence and/or the decoding to be modified according to the values of the physiological sensor.

WO 2006/054210 concerns a system and method for controlling the reproduction of audio signals via the monitoring of the state of relaxation of a person, wherein at least one physiological characteristic of the person is detected by means of a sensor, which characteristic assumes different values depending on different sleep phases of the person and wherein different sleep phases are recognized on the basis of at least one detected changing physiological characteristic by means of an analysis device. The speed for the reproduction of audio signals is changed on the basis of the at least one detected changing physiological characteristic and the reproduction of the audio signals is terminated by a control unit after a certain sleep phase of the person has commenced.

The present invention aims at obviating at least some of the disadvantages with known technology.

### SUMMARY OF THE INVENTION

These disadvantages are obviated by a portable device according to claim 1.

In this fashion a desired sleep, states can easily be induced in the user by a lightweight portable device which the user can carry everywhere. At the same time the actual sleep state of the user also affects the state of the portable device.

According to another aspect of the present invention the disadvantages of prior art are overcome by a system for inducing a target sleep state in a user comprising the features recited in claim 7.

In an aspect not forming part of the present invention, the disadvantages of known technology are overcome by an accessory unit.

It may be remarked here, that the accessory unit is especially suitable to be used together with the portable device described earlier and in a system for inducing a target sleep state in a user described above.

According to yet another aspect of the present invention, the disadvantages of known technology are overcome by a method according to claim 10.

It should be mentioned here, that the portable device described earlier is especially suitable for implementeing the method steps according to the present invention described above.

Finally and according to another aspect of the present invention, the disadvantages of known technology are overcome by a computer program according to claim 11.

It should also be remarked here, that the computer program is especially suited to execute the method steps stated above and to be executed in a portable device according to the Invention described earlier.

These and other advantages will be understood more clearly by reading the following detailed description below.

### BRIEF DESCRIPTION OF THE DRAWINGS (OPTIONAL)

Fig. 1 illustrates a system for relaxation and for inducing a sleep mode in the main unit and the accessory according to one embodiment of the present invention.
Fig. 2 illustrates a second embodiment of the relaxation apparatus and accessory.
Fig. 3 illustrates the steps of an embodiment of a method according to the present invention.

### DETAILED DESCRIPTION OF PREFERRED EMBODIMENTS

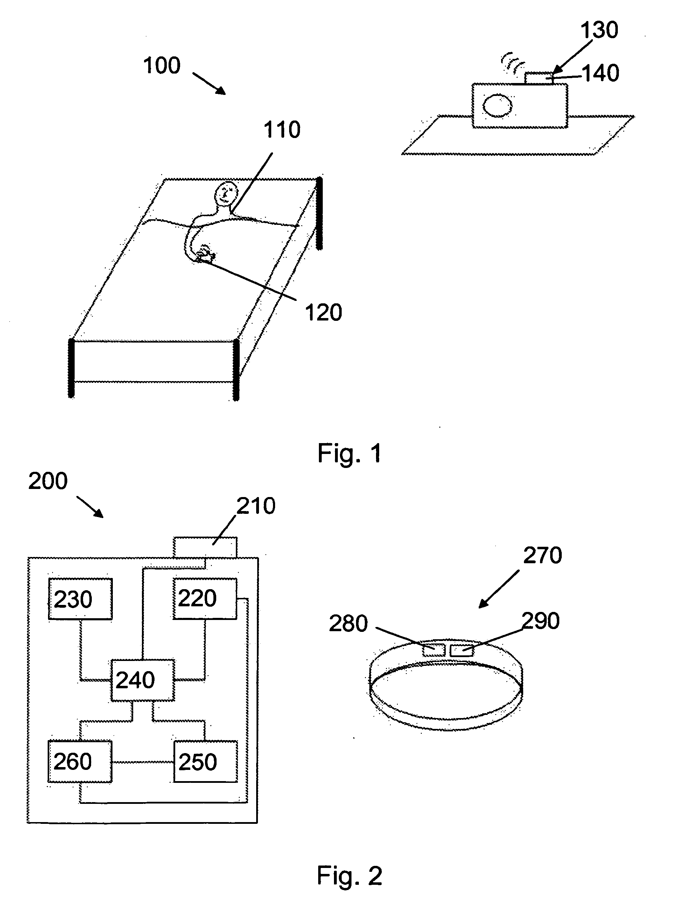

Fig. 1 illustrates a system 100 for relaxation and for inducing a sleep mode in a user 110 by means of a main unit 130 and an accessory unit 120 connected to the apparatus 130.

The user 110 in this example wears the accessory unit 120 in the form of a wristband on his or her wrist, where the sensor accessory registers the pulse rate of the user and sends the measurement data to the main unit 130. The accessory unit 120 may also be part of a wrist watch or comprise a Velcro band. The main point here is that the accessory unit 120 should have a firm contact with the user's wrist in order to be able to accurately measure the user's pulse rate.

The accessory unit 120 in this example is connected to the main unit 130 via a wireless link, such as for example a radio link, bluetooth, infrared, an IEEE 802series connection (.11, .15, .16 with sub standards a, b, g or n, for example) or some other wireless connection standard as long as it is suitable for transferring digital or analog data. However, the link between the wristband 120 and the main unit 130 may also be a wired link via a copper or an optical fiber cable or some other suitable wired connection.

It should be mentioned that when using the infrared link, a reasonable line of sight angle and distance should be maintained between the accessory unit 120 and the main unit 130 as is known to the person skilled in the area of infrared data communication.

A main unit 130 located a distance away from the sensor accessory is receiving the measurement signals or data from the accessory unit 120 via its receiver 140.

As a reaction to the received measurement signals or data which indicate the current physiological state of the user the main unit 130 either remains in the present mode of operation or changes to a sleep mode The sleep mode is usually activated if the heart rate of the user 110 falls below a certain predefined value, where the main unit 130 for example may switch to an analog or digital radio station with soothing or relaxing music. The main music may also play music or sound(s) stored in its memory (not shown) in case the main unit 130 is equipped with a memory.

Besides being a radio unit, the main unit 130, may also comprise any other device capable of radio or data communication and producing sounds or music. Also, the main unit 130 may, instead of being equipped solely with a receiver 140, also comprise a transmitter (not shown) for controlling the accessory unit 120 in order to control the measurement interval or the measurement method. Also a transmitter may be of use if the user desires to reach a certain target physiological state, such as relaxation, sleep but also other states, such as concentration or creativity so that the main unit 130 may signal to the accessory unit when to start the measurement.

It should be also mentioned that other means of measuring the physiological state of the user are possible, besides the measuring of the pulse rate. These measurements, such as measurements of the heart rate, breathing rate or other physiological parameters related to relaxation of the body may lead to a somewhat different construction of the accessory unit 120 and be provided as a torso band and/or be equipped with a microphone.

Apart from the variants above, the accessory unit 120 may be adapted to start measuring data indicative of the current physiological state of the user as soon as the user starts wearing it.

Turning now to Fig. 2, a portable main unit 200 and an associated accessory unit 270 according to a second embodiment of the present invention are schematically illustrated in the figure.

The main unit 200 comprises a portable terminal 200 equipped with a transceiver 210, at least one audio output unit 220, at least one display 230, a processing unit 240, a memory 250 and a user interface 260.

The term "portable" should be interpreted here as having such a weight and size that it can be carried by a human hand.

Its accessory unit 270 comprises at least one measurement sensor and a transmitter. Apart from these two elements, the accessory unit 270 also comprises a power source (not shown) in case it is operating on a wireless link with the portable terminal 200.

In the following, the function of the portable terminal 200 and its accessory 270 will be explained in detail.

By means of the transceiver 210, the portable terminal 200 receives measurement data which indicates the current physiological state of the user, where the measurement data is registered by the measurement sensors 280 of the accessory unit 270. This data may either be analog or digital signals, as preferred. Measurement data, such as the pulse rate of the user 110 is transferred by means of the transmitter 290 to the receiver 210 of the portable terminal 200. However, the portable terminal 270 may also comprise a receiver, which may be integrated together with the transmitter if, for example, changes of the sampling interval (if the registered pulse rate signals are to be digitized) need to be controlled by the terminal 200 or change to some other parameters is to be effectuated. A receiver may also be used if the measurement is to be initiated by the main unit 200. The processing unit 240 in this example may perform a digital-to-analog conversion of the measurement signals received from the accessory unit 270 if they are received in analog form and some further processing on them, such as amplification and filtering in order to be able to obtain accurate processing results in later processing stages.

The processing unit 240 in Fig. 2 may also be connected to a memory 250, where data in the form of different music or sounds as well as certain graphical diagrams or threshold values related to known physiological states of a human being are stored. The memory 250 may be an internal ROM (Read-Only-Memory) such as a FLASH-ROM or non-flashable ROM or a type of RAM (Random Access Memory), such as a memory card, hard disk, SIM-card or some built-in RAM-memory known to the skilled person. As far as the data, music and sounds in the memory 250 are concerned, it may either be pre-stored in the memory or downloaded and defined by the user of the portable terminal 200. Comparison of the signals received at the receiver 210 and processed in the processing unit 240 with the threshold values in the memory will give clues about the actual physiological state of the user of the accessory unit. Thereafter, the processing unit 240 may take appropriate action, such as playing music from the memory 250 over the audio output unit 220 of the portable terminal 200 and/or visualizing the received measurement data registered by the accessory unit 270 on the display 230 of the portable terminal 200. If the accessory unit 270 of the main unit 200 also comprises head phones, the music or sounds may be played via the head phones.

Also, the state of the portable unit 200 may be altered if an altered state of the user Is detected, such as decreasing pulse-, heart- or breathing rate. This altered state of the portable unit 200 may express itself through decreasing the volume of the music or the sounds and/or slowing down of the speed with which they are played. It may also result in decreasing the background lighting to the display of the portable unit 200.

Apart from the elements mentioned above, the main unit 200 also comprises a user interface 260 allowing the user to choose from different target physiological states he or she wishes to achieve, such as a station of relaxation, a sleeping state or states of concentration or heightened creativity to name a few examples. Using a display 230 Also the user may via the user interface 260 define the time interval for the measurements of data indicating his or her current physiological state. This presupposes however, that the accessory unit 270 additionally comprises a receiver (not shown) which is able to receive control information from the main unit 200 about the length of the measurement interval. However, the user may also set the measurement method to intermittent instead of continuous, as desired.

In case the portable terminal 200 also performs the function of a mobile terminal, the portable terminal 200 may change into a sleep mode, where incoming calls or messages are registered but no audio- or visual indication of their reception is signalled to the user.

Additionally, the accessory unit 270 may besides the sensor unit and the receiver 290 also comprise an audio output unit of its own (not shown) for outputting music or sound to the user. Such an accessory unit may for example comprise head phones In addition to a wristband described earlier. It also may comprise a headset, i.e. a headphone/microphone combination for measuring the breathing rate of the user. In this fashion, the accessory unit 270 may receive control signals from the main unit 200 for outputting music or sound files from the memory 250 of the main unit 200 depending on the measurement data sent to the main unit 200. The control signals from the main unit 200 may also comprise instructions to the accessory unit to adjust the music or sounds played to the user in order to reach a target physiological state described earlier.

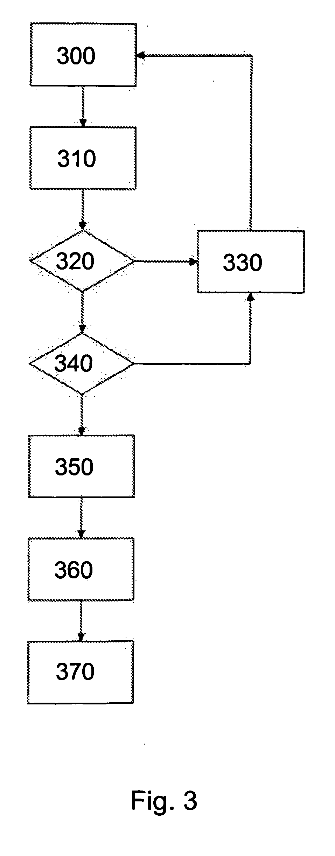

Turning now to Fig. 3 the steps performed according to a first embodiment of the present invention are illustrated.

At step 300 an accessory unit, such as the accessory unit 270 in Fig. 2 performs measurements of parameters which indicate the current physiological state of the user using the accessory unit. These parameters may range from pulse-, heart- or breathing rate to blood pressure and other parameters indicative of the physiological state of the user.

Thereafter, at step 310, the measured data is transmitted to a portable unit, such as the portable unit 200 in Fig. 2 and received there. It should be mentioned there that the communication between the accessory unit and the portable unit 200 may take place either via a wireless link, such as via Bluetooth, IEEE 802.serles (.11, .15, .16, and sub standards a, b, g, n), Infrared or by wired communication, such as via cable or optical fibers.

At step 320 a processing unit in the portable unit, such as the processing unit 240, analyses the received measurement data, if necessary converts the data to digital form by analog-to-digital conversion, performs amplification and filtering of the signal and compares the thus converted signal with a predefined first threshold value or a first graphical diagram. The predefined first threshold value or graphical diagram is related to a certain known physiological state of a human being, such as for example when he or she is relaxed or close to the sleeping state. It should be mentioned that the threshold value or graphical diagram may be predefined by the manufacturer of the portable terminal 200, but also be altered or downloaded by the user.

If the measurement signal received from the accessory unit 270 is above the predefined first threshold the processing unit may retrieve music or sounds from a memory of the portable unit at step 330, such as the memory 250 in Fig. 2 and instruct an audio output unit, such as the loudspeaker in Fig. 2 to output the music or sound audibly to the user.

The music or sounds stored in the memory of the portable unit may be chosen so that they will have a relaxing and possibly sleep inducing effect on the user. However, in other embodiments of the method (not shown), the music may be chosen to induce a state of heightened concentration or creativity in the individual.

However, if the measurement signal received is determined to be below the predefined first threshold value the processing unit compares the measurement signal to a predefined second threshold value, where the predefined second threshold value may, for example, indicate whether the user is asleep or not.

In the case that the received measurement value is above the predefined second threshold value, which may indicate that the user is relaxed but not sleeping, the processing unit 240, may at step 330 continue to play music or sounds from the memory 250 and additionally reduce the volume and/or speed at which the music or sounds are played (not shown). In this fashion a sleeping or an even more relaxed state in the user may be more easily induced.

If, however, the received measurement value through comparison with the predefined second threshold is determined to be below the predefined second threshold, the portable unit switches off the audio output unit 350 either by fading out the music or sounds or switching it off immediately. Hereby it may be possible to give the user of the portable unit the choice to define the fade-out period in advance or to set it to immediate turn-off.

At step 360 the portable unit 200 changes to sleep mode, which for example may comprise turning off its display, such as the display in Fig. 2 and other audio-visual functions.

Finally, the portable terminal comprises functionality for receiving telephone calls or electronic messages, the portable terminal may at step 370 switch to a state where calls or messages are received and registered, but where no audible or visual indications of the receipt are presented to the user.

While the embodiment of the method according to the present invention has been described for the case where the measurements are performed automatically on the user, the user may also define on the main unit that he wishes to reach a target state of relaxation or sleep or some other physiological state, such as heightened concentration or creativity. The main unit 200 may then signal to the accessory unit to initiate measurement of the parameters indicated above and use music or sounds to facilitate for the user to reach the desired target state.

It is also worthwhile mentioning that in the method described above, the measurements performed by the accessory unit may be continuous or intermittent, as preferred. The measurement interval may also be defined by the user.

Also, it should be pointed out that the operations performed in the method steps in Fig. 3 are well adapted to be executed by a computer program which is either stored in the memory a portable terminal, such as the memory 250 of the portable terminal 200 or may be downloaded to the memory 250 from an external source, such as a communication network.

It is understood that various modifications of the present invention may be performed by the skilled person who has studied the description and the accompanying drawings within the scope defined by the claims.

## Claims

1. A portable device (200) configured to induce a target sleep state in a user (110), whereby the device comprises: at least one receiver (140) configured to receive measurement data indicative of the current sleep state of the user (110), a processing unit (240) configured to process the received measurement data, at least one audio output unit (220) and a memory (250), the processing unit (240) being arranged to detect the current sleep state of the user (110) by comparing the processed received measurement data to predefined data in the memory (250) Indicative of well-defined sleep states, wherein the processing unit (240) is arranged to output audio data through the audio output unit (220) depending on the detected actual sleep state and to adjust the output of the audio data according to the target sleep state to be induced In the user (110), until the user (110) falls asleep, and then switching off the audio output unit (220) either by fading out music or sounds or switching it off immediately, **characterized in that** said portable device comprises functionality for receiving telephone calls or electronic messages and is arranged to switch to a state where calls or messages are received and registered, but where no audible or visual indications of the receipt are presented to the user when said audio output unit (220) has been switched off.

2. The device according to claim 1, further comprising a user (110) interface (260) via which a user (110) can define the above well-defined sleep state and select a desired target sleep state.

3. The device according to claims 1-2, further comprising a display (230) unit configured to display visual data indicative of the current sleep state of the user (110).

4. The device according to according to any of the claims 1 -3, further comprising a transmitter such that said portable device (200) may communicate in a wireless communication network,

5. The device according to according to any of the claims 1-4, wherein the measurement data Indicative of the sleep state of the user (110) is received continuously.

6. The device according to according to any of the claims 1-4, wherein the measurement data indicative of the sleep state of the user (110) is received intermittently.

7. A system for inducing a target sleep state in a user (110) comprising, **characterized in that** it comprises a portable device (200) according to claim 1 and an accessory unit (120, 270), the accessory unit (120, 270) comprising a sensing unit configured to measure data indicative of the current sleep state of the user (110) of the portable device (200) and a transmitter configured to transmit the measured data to the portable device (200).

8. The system according to according to claim 7 wherein the accessory unit (120, 270) further comprises a receiver (140) configured to receive control information from said portable device (200).

9. The system according to claim 8, wherein the control information comprises information on the parameters to be measured by the accessory unit (120, 270) and/or Information on the measurement interval.

10. A non-therapeutic method for inducing a target sleep state in a user (110) using a portable device (200), which comprises the steps; a) receiving measurement data indicative of the current sleep state of a user (110) of said portable device (200); b) processing the received measurement data; c) detecting the current sleep state of the user (114) by comparing the processed received measurement data to predefined data in the memory (250) indicative of well- defined sleep states; d) outputting audio data through an audio output unit (220); e) adjusting the output of the audio data according to the target sleep state to be induced in the user (110), until the user (110) falls asleep, and then switching off the audio output unit (220) either by fading out music or sounds or switching it off immediately, **characterized in that** said portable device (200) comprises functionality for receiving telephone calls or electronic messages, and said method comprises the step of f) switching the portable device (200) to a state where calls or messages are received and registered, but where no audible or visual indications of the receipt are presented to the user when said audio output unit (220) has been switched off.

11. A computer program configured to induce a target sleep state In a user (110) using a portable device (200), which computer program comprises instructions sets for: a) receiving measurement data indicative of the current sleep state of a user (110) of the portable device (200); b) processing the received measurement data; c) detecting the current sleep state of the user (110) by comparing the processed received measurement data to predefined data in the memory (250) indicative of well- defined sleep states; d) outputting audio data through an audio output unit (220) and, e) adjusting the output of the audio data according to the target sleep state to be induced In the user (110), until the user (110) falls asleep, and then switching off the audio output unit (220) either by fading out music or sounds or switching it off immediately, **characterized in that** said portable device (200) comprises functionality for receiving telephone calls or electronic messages, and said computer program comprises instructions sets for: switching the portable unit to a state where calls or messages are received and registered, but where no audible or visual indications of the receipt are presented to the user when said audio output unit (220) has been switched off.

## Patentansprüche

1. Tragbare Vorrichtung (200), welche ausgestaltet ist, um einen Ziel-Schlafzustand bei einem Benutzer (110) hervorzurufen, wobei die Vorrichtung umfasst: mindestens einen Empfänger (140), welcher ausgestaltet ist, um Messdaten zu erfassen, welche den aktuellen Schlafzustand des Benutzers (110) anzeigen, eine Verarbeitungseinheit (240), welche ausgestaltet ist, um die erfassten Messdaten zu verarbeiten, mindestens eine Audio-Ausgangseinheit (220) und einen Speicher (250), wobei die Verarbeitungseinheit (240) eingerichtet ist, um den aktuellen Schlafzustand des Benutzers (110) zu erfassen, indem die verarbeiteten erfassten Messdaten mit vordefinierten Daten in dem Speicher (250), welche gut definierte Schlafzustände anzeigen, verglichen werden, wobei die Verarbeitungseinheit (240) eingerichtet ist, um Audiodaten über die Audio-Ausgangseinheit (220) abhängig von dem erfassten aktuellen Schlafzustand auszugeben und die Ausgabe der Audiodaten gemäß dem Ziel-Schlafzustand, welcher bei dem Benutzer (110) hervorzurufen ist, einzustellen, bis der Benutzer (110) in einen Schlaf fällt, und um dann die Audio-Ausgangseinheit (220) auszuschalten, indem entweder Musik oder Geräusche ausgeblendet werden oder sie sofort ausgeschaltet wird, **dadurch gekennzeichnet, dass** die tragbare Vorrichtung eine Funktionalität umfasst, um Telefonanrufe oder elektronische Nachrichten zu empfangen, und eingerichtet ist, um in einen Zustand zu schalten, in welchem Anrufe oder Nachrichten empfangen und aufgezeichnet werden, in welchem aber keine hörbaren oder sichtbaren Anzeigen des Empfangs dem Benutzer präsentiert werden, wenn die Audio-Ausgangseinheit (220) ausgeschaltet worden ist.

2. Vorrichtung nach Anspruch 1, darüber hinaus eine Benutzer (110)-Schnittstelle (260) umfassend, mittels welcher ein Benutzer (110) den gut bekannten Schlafzustand definieren und einen erwünschten Ziel-Schlafzustand auswählen kann.

3. Vorrichtung nach Anspruch 1 oder 2, darüber hinaus eine Anzeige (230)-Einheit umfassend, welche ausgestaltet ist, um visuell Daten anzuzeigen, welche den aktuellen Schlafzustand des Benutzers (110) anzeigen.

4. Vorrichtung nach einem der Ansprüche 1-3, darüber hinaus einen Sender umfassend, so dass die tragbare Vorrichtung (200) in einem drahtlosen Kommunikationsnetz kommunizieren kann.

5. Vorrichtung nach einem der Ansprüche 1-4, **dadurch gekennzeichnet, dass** die Messdaten, welche den Schlafzustand des Benutzers (110) anzeigen, kontinuierlich erfasst werden.

6. Vorrichtung nach einem der Ansprüche 1-4, **dadurch gekennzeichnet, dass** die Messdaten, welche den Schlafzustand des Benutzers (110) anzeigen, mit Unterbrechungen erfasst werden.

7. System zum Anzeigen eines Ziel-Schlafzustands bei einem Benutzer (110), **dadurch gekennzeichnet, dass** es eine tragbare Vorrichtung (200) nach Anspruch 1 und eine Hilfseinheit (120, 270) umfasst, wobei die Hilfseinheit (120, 270) umfasst eine Erfassungseinheit, welche ausgestaltet ist, um Daten zu erfassen, welche den aktuellen Schlafzustand des Benutzers (110) der tragbaren Vorrichtung (200) anzeigen, und einen Sender, welcher ausgestaltet ist, um die gemessenen Daten zu der tragbaren Vorrichtung (200) zu senden.

8. System nach Anspruch 7, **dadurch gekennzeichnet, dass** die Hilfseinheit (120, 270) darüber hinaus einen Empfänger (140) umfasst, welcher ausgestaltet ist, um eine Steuerinformation von der tragbaren Vorrichtung (200) zu empfangen.

9. System nach Anspruch 8, **dadurch gekennzeichnet, dass** die Steuerinformation eine Information bezüglich der Parameter, welche durch die Hilfseinheit (120, 270) zu messen sind, und/oder eine Information bezüglich des Messintervalls umfasst.

10. Nicht therapeutisches Verfahren, um einen Ziel-Schlafzustand bei einem Benutzer (110) hervorzurufen, welcher eine tragbare Vorrichtung (200) verwendet, welches die Schritte umfasst: a) Empfangen von Messdaten, welche den aktuellen Schlafzustand eines Benutzers (110) der tragbaren Vorrichtung (200) anzeigen; b) Verarbeiten der erfassten Messdaten; c) Erfassen des aktuellen Schlafzustands des Benutzers (110), indem die verarbeiteten erfassten Messdaten mit vordefinierten Daten in dem Speicher (250), welche gut definierte Schlafzustände anzeigen, verglichen werden; d) Ausgeben von Audiodaten über eine Audio-Ausgangseinheit (220); e) Einstellen der Ausgabe der Audiodaten entsprechend dem Ziel-Schlafzustand, welcher bei dem Benutzer (110) hervorzurufen ist, bis der Benutzer (110) in einen Schlaf fällt, und dann Ausschalten der Audio-Ausgangseinheit (220), indem entweder Musik oder Geräusche ausgeblendet werden oder sie sofort ausgeschaltet wird, **dadurch gekennzeichnet, dass** die tragbare Vorrichtung (200) eine Funktionalität umfasst, um Telefonanrufe oder elektronische Nachrichten zu empfangen, und dass das Verfahren den Schritt umfasst f) Schalten der tragbaren Vorrichtung (200) in einen Zustand, in welchem Anrufe oder Nachrichten empfangen und aufgezeichnet werden, in welchem aber keine hörbaren oder sichtbaren Anzeigen des Empfangs dem Benutzer präsentiert werden, wenn die Audio-Ausgangseinheit (220) ausgeschaltet worden ist.

11. Computerprogrammprodukt, welches ausgestaltet ist, um einen Ziel-Schlafzustand bei einem Benutzer (110) hervorzurufen, welcher eine tragbare Vorrichtung (200) verwendet, wobei das Computerprogramm Anweisungssätze umfasst für: a) Empfangen von Messdaten, welche den aktuellen Schlafzustand eines Benutzers (110) der tragbaren Vorrichtung (200) anzeigen; b) Verarbeiten der erfassten Messdaten; c) Erfassen des aktuellen Schlafzustands des Benutzers (110), indem die verarbeiteten erfassten Messdaten mit vordefinierten Daten in dem Speicher (250), welche gut definierte Schlafzustände anzeigen, verglichen werden; d) Ausgeben von Audiodaten über eine Audio-Ausgangseinheit (220); und e) Einstellen der Ausgabe der Audiodaten entsprechend dem Ziel-Schlafzustand, welcher bei dem Benutzer (110) hervorzurufen ist, bis der Benutzer (110) in einen Schlaf fällt, und dann Ausschalten der Audio-Ausgangseinheit (220), indem entweder Musik oder Geräusche ausgeblendet werden oder sie sofort ausgeschaltet wird, **dadurch gekennzeichnet, dass** die tragbare Vorrichtung (200) eine Funktionalität umfasst, um Telefonanrufe oder elektronische Nachrichten zu empfangen, und dass das Computerprogramm Anweisungssätze umfasst für: Schalten der tragbaren Vorrichtung in einen Zustand, in welchem Anrufe oder Nachrichten empfangen und aufgezeichnet werden, in welchem aber keine hörbaren oder sichtbaren Anzeigen des Empfangs dem Benutzer präsentiert werden, wenn die Audio-Ausgangseinheit (220) ausgeschaltet worden ist.

## Revendications

1. Dispositif portable (200) configuré pour induire un état de sommeil cible chez un utilisateur (110), moyennant quoi le dispositif comprend : au moins un récepteur (140) configuré pour recevoir des données de mesure indicatives de l'état de sommeil actuel de l'utilisateur (110), une unité de traitement (240) configurée pour traiter les données de mesure reçues, au moins une unité de sortie audio (220) et une mémoire (250), l'unité de traitement (240) étant agencée pour détecter l'état de sommeil actuel de l'utilisateur (110) en comparant les données de mesure reçues traitées à des données prédéfinies dans la mémoire (250) indicatives d'états de sommeil bien définis, dans lequel l'unité de traitement (240) est agencée pour délivrer des données audio par l'intermédiaire de l'unité de sortie audio (220) en fonction de l'état de sommeil réel détecté et pour ajuster la sortie des données audio en fonction de l'état de sommeil cible à induire chez l'utilisateur (110), jusqu'à ce que l'utilisateur (110) s'endorme, et désactiver ensuite l'unité de sortie audio (220) soit en atténuant la musique ou les sons, soit en la coupant immédiatement, ledit dispositif portable étant **caractérisé en ce qu'**il comprend une fonctionnalité pour recevoir des appels téléphoniques ou des messages électroniques et est agencé pour commuter dans un état dans lequel les appels ou les messages sont reçus et enregistrés, mais dans lequel aucune indication audible ou visuelle de la réception n'est présentée à l'utilisateur lorsque ladite unité de sortie audio (220) a été désactivée.

2. Dispositif selon la revendication 1, comprenant en outre une interface (260) d'utilisateur (110) par l'intermédiaire de laquelle un utilisateur (110) peut définir l'état de sommeil bien défini ci-dessus et sélectionner un état de sommeil cible souhaité.

3. Dispositif selon les revendications 1 et 2, comprenant en outre une unité d'affichage (230) configurée pour afficher des données visuelles indicatives de l'état de sommeil actuel de l'utilisateur (110).

4. Dispositif selon l'une quelconque des revendications 1 à 3, comprenant en outre un émetteur de sorte que ledit dispositif portable (200) puisse communiquer dans un réseau de communication sans fil.

5. Dispositif selon l'une quelconque des revendications 1 à 4, dans lequel les données de mesure indicatives de l'état de sommeil de l'utilisateur (110) sont reçues continûment.

6. Dispositif selon l'une quelconque des revendications 1 à 4, dans lequel les données de mesure indicatives de l'état de sommeil de l'utilisateur (110) sont reçues par intermittence.

7. Système pour induire un état de sommeil cible chez un utilisateur (110), **caractérisé en ce qu'**il comprend un dispositif portable (200) selon la revendication 1 et une unité d'accessoires (120, 270), l'unité d'accessoires (120, 270) comprenant une unité de détection configurée pour mesurer des données indicatives de l'état de sommeil actuel de l'utilisateur (110) du dispositif portable (200) et un émetteur configuré pour transmettre les données mesurées au dispositif portable (200).

8. Système selon la revendication 7, dans lequel l'unité d'accessoires (120, 270) comprend en outre un récepteur (140) configuré pour recevoir des informations de commande dudit dispositif portable (200).

9. Système selon la revendication 8, dans lequel les informations de commande comprennent des informations concernant les paramètres à mesurer par l'unité d'accessoires (120, 270) et/ou des informations concernant l'intervalle de mesure.

10. Procédé non thérapeutique pour induire un état de sommeil cible chez un utilisateur (110) en utilisant un dispositif portable (200), qui comprend les étapes : a) de réception de données de mesure indicatives de l'état de sommeil actuel d'un utilisateur (110) dudit dispositif portable (200) ; b) de traitement des données de mesure reçues ; c) de détection de l'état de sommeil actuel de l'utilisateur (110) en comparant les données de mesure reçues traitées à des données prédéfinies dans la mémoire (250) indicatives d'états de sommeil bien définis ; d) de sortie de données audio par l'intermédiaire d'une unité de sortie audio (220) ; e) d'ajustement de la sortie des données audio en fonction de l'état de sommeil cible à induire chez l'utilisateur (110), jusqu'à ce que l'utilisateur (110) s'endorme, et de désactivation ensuite de l'unité de sortie audio (220) soit en atténuant la musique ou les sons, soit en la coupant immédiatement, **caractérisé en ce que** ledit dispositif portable (200) comprend une fonctionnalité pour recevoir des appels téléphoniques ou des messages électroniques, et ledit procédé comprend l'étape f) de commutation du dispositif portable (200) dans un état dans lequel les appels ou les messages sont reçus et enregistrés, mais dans lequel aucune indication audible ou visuelle de la réception n'est présentée à l'utilisateur lorsque ladite unité de sortie audio (220) a été désactivée.

11. Programme d'ordinateur configuré pour induire un état de sommeil cible chez un utilisateur (110) en utilisant un dispositif portable (200), lequel programme d'ordinateur comprend des jeux d'instructions pour a) recevoir des données de mesure indicatives de l'état de sommeil actuel d'un utilisateur (110) du dispositif portable (200) ; b) traiter les données de mesure reçues ; c) détecter l'état de sommeil actuel de l'utilisateur (110) en comparant les données de mesure reçues traitées à des données prédéfinies dans la mémoire (250) indicatives d'états de sommeil bien définis ; d) sortir des données audio par l'intermédiaire d'une unité de sortie audio (220) ; et e) ajuster la sortie des données audio en fonction de l'état de sommeil cible à induire chez l'utilisateur (110), jusqu'à ce que l'utilisateur (110) s'endorme, et désactiver ensuite l'unité de sortie audio (220) soit en atténuant la musique ou les sons, soit en la coupant immédiatement, **caractérisé en ce que** ledit dispositif portable (200) comprend une fonctionnalité pour recevoir des appels téléphoniques ou des messages électroniques, et ledit programme d'ordinateur comprend des jeux d'instructions pour commuter l'unité portable dans un état dans lequel les appels ou les messages sont reçus et enregistrés, mais dans lequel aucune indication audible ou visuelle de la réception n'est présentée à l'utilisateur lorsque ladite unité de sortie audio (220) a été désactivée.
